# FASCICULE DE BREVET EUROPEEN

(11) **EP 0 880 963 B1**
(45) Date de publication et mention de la délivrance du brevet: **30.01.2002**
(21) Numéro de dépôt: 98400991.0
(22) Date de dépôt: 23.04.1998
(51) Int. Cl.: A61K 7/48, A61K 47/32, A61K 47/34

(54) **Association d'un rétinoide avec un polymère polyaminé**
Mischung eines Retinoids mit einem Polymamin-Polymeren
Association of a retionoid with a polyamino-polymer

(30) Priorité: 28.05.1997 FR 9706533
(43) Date de publication de la demande: 02.12.1998
(73) Titulaire: L'OREAL, 75008 Paris (FR)
(72) Inventeur: Nguyen, Quang Lan, 92160 Antony (FR); Boussoira, Boudiaf, 75020 Paris (FR); Prince, Stéphanie, 75014 Paris (FR); Giacomoni, Paolo, 91400 Orsay (FR)
(74) Mandataire: Tezier Herman, Béatrice

(56) Documents cités:
- EP-A- 0 260 186
- WO-A-96/15810
- A. R. VIGUERA ET AL.: "A water-soluble polylysine-retinaldehyde Schiff base. Stability in aqueous and nonaqueous environments." J. BIOL. CHEM., vol. 265, no. 5, 1990, pages 2527-2532, XP002057413
- BASE DE DONN ES: "CHEMICAL ABSTRACTS" (SERVEUR: STN); abr.114: 128 811, Colombus OH, USA; & JP 02 261 534 A (SHISEIDO CO.) 24 Octobre 1990 XP002057414

## Description

La présente invention a pour objet de nouvelles compositions cosmétiques et/ou dermatologiques, en particulier des compositions destinées au soin de la peau et comprenant au moins un composé de la famille des rétinoïdes. Plus particulièrement, l'invention a pour objet des compositions stables comprenant au moins un composé de la famille des rétinoïdes et au moins un polymère polyaminé.

Les compositions cosmétiques et/ou dermatologiques à base de rétinoïdes ont connu un important développement au cours de ces dernières années. Parmi les rétinoïdes, l'utilisation de l'acide rétinoïque dans des compositions pour le traitement de l'acné est bien connue.

Toutefois, il est apparu que d'autres dérivés de la famille des rétinoïdes présentent un intérêt, à la fois pour le traitement de l'acné, et également pour le soin de la peau, en particulier pour limiter, voire supprimer, les effets du vieillissement sur la peau : les rides, l'aspect parcheminé, le jaunissement, la perte d'élasticité, la rugosité, la sécheresse, l'apparition de tâches sont les manifestations habituelles du vieillissement de la peau. Ces manifestations sont d'autant plus accentuées que la peau a été fréquemment exposée au soleil ou est particulièrement sensible à l'exposition aux rayonnements UV.

Ainsi, les effets du vieillissement intrinsèque de la peau (lié à l'âge) et du photo-vieillissement (dû à l'exposition au soleil) peuvent se cumuler. Les manifestations du vieillissement apparaissent habituellement à un âge avancé, toutefois, leur prévention doit être entreprise dès le début de la vie d'adulte par des soins appropriés.

Le traitement de la peau par des dérivés de la famille des rétinoïdes fait partie de ces soins préventifs ou curatifs des manifestations du vieillissement que sont : les rides, la peau parcheminée, le jaunissement, la perte d'élasticité, la rugosité, la sécheresse, les tâches.

Parmi les dérivés de la famille des rétinoïdes, le rétinol, également connu sous le nom de vitamine A et les dérivés estérifiés du rétinol présentent un intérêt tout particulier. En effet, le rétinol est un constituant endogène naturel de l'organisme humain. Il est bien toléré en application sur la peau jusqu'à des taux beaucoup plus élevés que l'acide rétinoïque. Les esters du rétinol sont convertis en rétinol par l'organisme humain.

Toutefois, lorsqu'ils sont introduits dans une composition cosmétique ou dermatologique destinée à une application topique, le rétinol et ses esters se dégradent rapidement, sous l'effet de la lumière, de l'oxygène, des ions métalliques, des agents oxydants, de l'eau ou en particulier sous l'effet d'une élévation de température. La dégradation thermique du rétinol a fait l'objet d'une étude publiée dans J. Soc. Cosm. Chem. 46, 191 - 198 (July-August 1995).

On connaît différentes associations du rétinol ou d'autres dérivés de la famille des rétinoïdes avec des anti-oxydants, les rétinoïdes présentant une stabilité améliorée au sein de ces associations :

Le document W093/00085 décrit des émulsions E/H comprenant du rétinol et un système stabilisant constitué d'un agent chélatant, comme par exemple l'EDTA, et un anti-oxydant qui peut être soit un anti-oxydant liposoluble, comme le butyl hydroxy toluène (BHT) ou la vitamine E, soit un antioxydant hydrosoluble comme la vitamine C.

Selon ce document, on peut également préparer des émulsion E/H contenant du rétinol stabilisé par un système constitué d'un anti-oxydant liposoluble et d'un anti-oxydant hydrosoluble.

Le document EP-608433 décrit des compositions contenant du rétinol et un agent stabilisant choisi parmi les agents chélatants et les polysaccharides, les huiles d'indice d'iode supérieur à 70, les polyéthylène (propylène) glycols, les sels d'acides hydroxycarboxyliques, les sels d'acides aminés neutres, les anti-oxydants liposolubles associés à l'EDTA et à une benzophénone, les anti-oxydants liposolubles associés à un composé acide et à une benzophénone, les dérivés de cyclodextrine dans lesquels est inclus un anti-oxydant ou un filtre UV, le butanediol et/ou les anti-oxydants liposolubles, les dérivés de benzophénone hydrosolubles, les acides aminés basiques et leurs sels les acides aminés acides et leurs sels, les huiles polaires, les argiles minérales hydrophiles.

On connaît par le document EP-209509 l'utilisation de certains composés polyaminés comme anti-oxydants.

Toutefois, aucun des composés de l'art antérieur, qu'il soit décrit comme anti-oxydant ou plus spécifiquement comme stabilisateur de rétinoïde, ne permet d'obtenir une stabilisation satisfaisante des rétinoïdes.

Or, la demanderesse a découvert avec étonnement que l'association d'un rétinoïde choisi parmi la vitamine A (ou rétinol) et les précurseurs bio-convertibles de la vitamine A, avec certains polymères polyaminés, permettait d'éviter la dégradation, en particulier la dégradation thermique, de ces rétinoïdes. Ainsi, ces rétinoïdes, peuvent être introduits dans des compositions cosmétiques et/ou dermatologiques, être stockés pendant plusieurs mois, sans voir se dégrader leur efficacité.

L'invention a donc pour objet une composition cosmétique et/ou dermatologique adaptée à une application sur la peau du corps ou les cheveux, comprenant un support physiologiquement acceptable et au moins un rétinoïde choisi parmi la vitamine A et les précurseurs bio-convertibles de la vitamine A, caractérisée par le fait qu'elle comprend au moins un polymère polyaminé, choisi parmi :
(A) une polyalkylène polyamine ou un de ses dérivés choisi parmi :
   (i) les polyalkylène polyamines ;
   (ii) les dérivés alkylés de polyalkylène polyamines (A)(i) ;
   (iii) les produits d'addition d'acides alkylcarboxyliques sur les polyalkylènepolyamines (A)(i) ;
   (iv) les produits d'addition de cétones et d'aldéhydes sur les polyalkylènepolyamines (A)(i) ;
   (v) les produits d'addition d'isocyanates et d'isothiocyanates sur les polyalkylènepolyamines (A)(i) ;
   (vi) les produits d'addition d'oxyde d'alkylène ou de polymères blocs polyoxyde d'alkylène sur les polyalkylènepolyamines (A)(i) ;
   (vii) les dérivés quaternisés de polyalkylène polyamines (A)(i) ;
   (viii) les produits d'addition d'une silicone sur les polyalkylènepolyamines (A)(i) ;
   (ix) un copolymère d'acide dicarboxylique et de polyalkylène polyamines (A)(i) ;
(B) les polyvinylimidazoles ;
(C) les polyvinylpyridines ;
(D) les produits d'addition de monomères 1-vinylimidazole de formule (I): 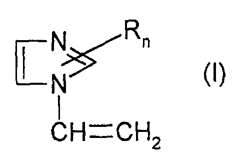 dans laquelle les radicaux R, identiques ou différents, représentent H, ou un radical alkyle en C₁-C₆, saturé ou insaturé, linéaire ou cyclique
   n est un entier allant de 1 à 3,
   avec les polyalkylènepolyamines (A)(i) à (A)(ix) ;
(E) les dérivés réticulés des polymères (A)(i) à (A)(ix), (B) (C) et (D).

Par vitamine A, on entend le rétinol de type all-trans ou de type 13-cis.

L'expression précurseur bio-convertible de la vitamine A désigne tout composé susceptible d'être converti en vitamine A par l'organisme humain. Parmi ces composés, on peut citer les esters de rétinol, en particulier les esters en C₁-C₃₀. Parmi cette famille de composés, l'invention concerne plus particulièrement les esters en C₁-C₆ qui sont très rapidement dégradés en rétinol par l'organisme humain.

L'invention a encore pour objet l'utilisation de la composition précédemment décrite pour la fabrication d'une préparation destinée au traitement dermatologique de lutte contre, et/ou de prévention de, l'irritation, l'inflammation, l'immunosuppression et/ou l'acné.

L'invention a, en outre, pour objet un procédé de traitement cosmétique consistant à lutter contre les signes du viellissement, particulièrement les signes du vieillissement induits par la photoperoxydation, en particulier la photoperoxydation du squalène et/ou du collagène, par application topique sur la peau et/ou le cuir chevelu et/ou les cheveux d'une composition comprenant au moins un rétinoïde choisi parmi la vitamine A (ou rétinol) et les précurseurs bio-convertibles de la vitamine A et au moins un polymère polyaminé selon l'invention, les méthodes de traitement thérapeutique du corps humain étant exclues.

Elle a aussi pour objet l'utilisation d'une telle composition cosmétique pour lutter contre et/ou prévenir les signes du vieillissement photoinduits de la peau et/ou des cheveux, les méthodes de traitement thérapeutique du corps humain étant exclues.

D'autres caractéristiques, aspects et avantages de la présente invention apparaîtront à la lecture de la description détaillée qui va suivre.

Les polymères polyaminés utilisables dans la présente invention peuvent se trouver sous la forme de polymère linéaire, de polymère hyperbranché ou de dendrimère.

Les polymères hyperbranchés sont des contructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications ; une couche de chaines terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%. On peut faire réagir un groupement terminal T sur le polymère hyperbranché pour obtenir une fonctionalité particulière en extrémité de chaines.

De tels polymères sont décrits en particulier dans B.I.Voit, Acta Polymer., 46, 87-99 (1995) ; EP-682059 ; WO-9614346 ; WO-9614345 ; WO-9612754.

Plusieurs polymères hyperbranchés peuvent être associés entre eux, par une liaison covalente ou un autre type de liaison, par l'intermédiaire de leurs groupes terminaux. De tels polymères dits pontés ou « bridged » entrent dans la définition des polymères hyperbranchés selon la présente invention.

Les dendrimères sont des polymères et oligoméres hautement ramifiés, également connus, ayant une structure chimique bien définie, et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la N^{ième} génération sont les groupes fonctionnels terminaux des fuseaux de la Nième génération ou génération terminale. De tels polymères sont décrits en particulier dans D.A.Tomalia, A.M.Naylor et W.A.Goddard III, *Angewandte Chemie,* Int.Ed.Engl.29, 138-175 (1990) ; C.J.Hawker et J.M.J.Frechet, *J.Am.Chem.Soc*., 112, 7638 (1990) ; B.I.Voit, Acta Polymer., 46, 87-99 (1995) ; N.Ardoin et D.Astruc, Bull. Soc. Chim. Fr. 132, 875-909 (1995).

On peut également définir plus particulièrement les dendrimères par la formule (Dl) suivante :

C[A₁B₁(A₂B₂(...(Aₙ₋₁ Bₙ₋₁(AₙBₙ(T)rₙ)rₙ₋₁)rₙ₋₂...)r₂)r₁]s (DI)

dans laquelle :
- C représente le coeur, relié par un nombre s de fonctionnalités à s fuseaux A₁B₁ par l'intermédiaire des groupements A₁ ;
- s est un nombre entier supérieur ou égal à 1 et inférieur ou égal au nombre de fonctionnalités de C ;
- l'indice i (i=1, 2.....n) est un nombre entier qui désigne la génération de chaque fuseau ;
- r₁ (i=1, 2.....n-1) représente le nombre de fonctionnalités du groupement B, appartenant au fuseau (AᵢBᵢ), ri étant un entier supérieur ou égal à 2
- pour chaque fuseau (AᵢBᵢ) (i=1, 2.....n), le groupement B, est relié à r, groupements Aᵢ₊₁ d'un fuseau (Aᵢ₊₁Bᵢ₊₁) ;
- chaque groupement A₁ (i ≥ 2) est relié à un seul groupement Bᵢ₋₁ du fuseau (Aᵢ₊₁Bᵢ₋₁) ;
- le fuseau de n^{ième} génération AₙBₙ est chimiquement lié à un nombre rₙ de groupes terminaux T, rₙ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines α et ε de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On peut se reporter aux documents suivants dans lesquels sont décrits des dendrimères comportant des groupements fonctionnels amines, le contenu de ces documents étant incorporé par référence dans la présente description US-4,694,064 ; US-4,631,337 ; WO-A-9502008 ; WO-A-9314147 ; US-4,360,646 ; Proc. Natl. Acad. Sci. USA, 85, 5409-5413 (1988).

Les polymères hyperbranchés et les dendrimères à groupes fonctionnels aminés peuvent également être constitués d'un coeur et de générations d'unités de base, monomères ou fuseaux, de toutes natures, sur lesquels un groupe terminal T porteur d'une fonction amine a été greffé.

On va décrire de façon plus approfondie les polymères polyaminés (A)(i) à (A)(ix), (B), (C), (D) et (E) de l'invention :
(A) (i) les polyalkylène polyamines utilisés préférentiellement selon l'invention sont des polymères contenant de 7 à 20000 motifs de répétition. De préférence, on choisit des polyalkylène polyamines comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.
   Ces polymères comprennent les motifs répétitifs suivants : 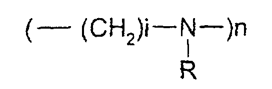 dans lequel:
   i représente un entier supérieur ou égal à 2, préférentiellement i=2 ;
   n représente un entier
   R représente H ou un motif 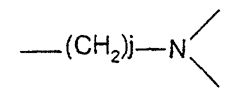 dans lequel j représente un entier supérieur ou égal à 2, préférentiellement j=2 ;

   Parmi les produits de la famille des polyalkylène polyamines, appelés aussi polyaziridines, on peut citer en particulier :
   La polyéthylèneimine, qui est un polymère hyperbranché bien connu de l'homme du métier. Au sujet de la polyéthylèneimine on peut se reporter en particulier aux documents : « KIRK-OTHMER ENCYCLOPEDIA OF CHEMICAL TECHNOLOGY », 3^{ème} édition, vol.20, 1982, p214-216 et "Polyéthylèneimine Prospective Application" H.N. Feigenbaum, Cosmetic & Toiletries, 108, 1993, p 73. La polyéthylèneimine est disponible commercialement auprès de la société BASF sous les noms de marques LUPASOL ou POLYIMIN ; la polyéthylèneimine est habituellement comprise dans une gamme de poids moléculaire moyen allant de 500 à 2.000.000 ;
   On connait aussi des polyéthylèneimines et des polypropylèneimines sous la forme de dendrimères, fabriqués par la société DSM. Les demandes de brevet WO 95/02008 et WO 93/14147 dont le contenu est incorporé à la présente demande par référence, décrivent des polyalkylènepolyamines de la famille des dendrimères ainsi qu'un procédé pour leur préparation.
(A) (ii) les dérivés alkylés de polyalkylène polyamine sont des produits bien connus de l'homme du métier. Ils sont obtenus de façon connue par alkylation, en milieu aqueux ou alcoolique, en présence d'un agent alkylant, de préférence en présence de NaOH, de KOH ou de carbonate, à des températures allant de préférence de 40°C à 130°C. L'agent alkylant peut être choisi par exemple parmi des dérivés sulfate d'alkyle ou halogénure d'alkyle en C₁-C₈, comme par exemple le diméthylsulfate, le diéthylsulfate, le bromure de butyle, le bromure d'hexyle, le bromure de 2-éthylhexyle, le bromure de n-octyle ou les chlorures correspondants. On peut par exemple se reporter à DE-3743744 qui décrit la préparation de tels produits, et dont le contenu est incorporé par référence au contenu de la présente demande.
(A) (iii) Les produits d'addition d'acides alkylcarboxyliques sur des polyalkylène polyamines sont des produits connus de l'homme du métier et dont la préparation est décrite par exemple dans les demandes de brevet WO 94/14873 ; WO 94/20681 ; WO 94/12560 dont le contenu est incorporé à la présente demande par référence. L'addition d'acides alkylcarboxyliques sur des polyalkylènepolyamines peut être effectuée en faisant réagir de façon connue un acide, un amide, un ester, un halogénure d'acide sur le polymère polyalkylènepolyamine.
   Les produits d'addition d'acides alkylcarboxyliques sur des polyalkylènepolyamines, peuvent être par exemple les produits d'addition d'acides alkylcarboxyliques en C₂-C₃₀, saturés ou insaturés, linéaires ou ramifiés, sur une polyéthylèneimine. Parmi les acides carboxyliques utilisables, on peut citer par exemple, l'acide acétique, l'acide propionique, l'acide butyrique, l'acide 2-éthylhexanoïque, l'acide benzoïque, l'acide laurique, l'acide myristique, l'acide palmitique, l'acide stéarique, l'acide oléique, l'acide linoléique, l'acide arachidonique, l'acide béhénique, ainsi que les mélanges de corps gras, comme par exemple les mélanges d'esters gras disponibles sous forme de produits naturels, et parmi lesquels on peut citer : l'huile de coco, l'huile de soja, l'huile de lin, l'huile de colza.
(A) (iv) les produits d'addition de cétones et d'aldéhydes sur les polyalkylènepolyamines (A)(i) peuvent être préparés par des procédés connus de l'homme du métier et conduisent à l'obtention de motifs α-hydroxyamine ;
(A) (v) les produits d'addition d'isocyanates et d'isothiocyanates sur les polyalkylènepolyamines (A)(i) peuvent être préparés par des procédés connus de l'homme du métier et conduisent à l'obtention de motifs urée et thio-urée ;
(A) (vi) les produits d'addition d'oxyde d'alkylène ou de polymères blocs polyoxyde d'alkylène sur les polyalkylènepolyamines (A)(i) peuvent être préparés par des procédés connus de l'homme du métier ; on peut se reporter par exemple aux documents EP-541018 et US-4,144,123, dans lesquels sont décrites de telles molécules ; des dérivés de polyéthylèneimine éthoxylés sont disponibles commercialement sous le nom de marque : LUPASOL 61(BASF)
(A) (vii) les dérivés quaternisés de polyalkylène polyamines (A)(i) peuvent être préparés par des procédés connus de l'homme du métier ;
(A) (viii) les produits d'addition d'une silicone sur les polyalkylènepolyamines (A)(i) sont par exemple des polyéthylèneimines greffées par des motifs polydiméthylsiloxane dont la préparation est décrite dans le document US-5,556,616 et commercialisées par la société MAC INTYRE sous le nom de marque MACKAMER PAVS ;
(A) (ix) les copolymères d'acide dicarboxylique et de polyalkylène polyamines (A)(i) peuvent être préparés par polycondensation d'acides dicarboxyliques avec des polyalkylène polyamines.
   Parmi les acides dicarboxyliques pouvant être utilisés pour la préparation des polyamidoamines on peut citer les acides dicarboxyliques en C₂ à C₁₀, comme par exemple l'acide oxalique, l'acide malonique, l'acide itaconique, l'acide succinique, l'acide maléique, l'acide adipique, l'acide glutarique, l'acide sébacique, l'acide téréphtalique, l'acide orthophtalique, ainsi que leurs mélanges.
   Les polyalkylène polyamines utilisées pour la préparation des polyamidoamines sont avantageusement choisies parmi celles ayant de 3 à 10 atomes d'azote, comme par exemple la diéthylène tri-amine, la triéthylène tétramine, la dipropylène tri-amine, la tripropylène tétramine, la di-hexaméthylène triamine, l'amino propyléthylène di-amine, la bis-aminopropyléthylène di-amine ainsi que leurs mélanges. On peut également utiliser des polyéthylène imines telles que décrites ci-dessus pour la préparation de polyamidoamines.
   De tels composés sont décrits par exemple dans les documents : US 4,144,423 et WO 94/29422.
(B) le terme polyvinylimidazole comprend les homopolymères et les copolymères de polyvinylimidazole (PVI) obtenus par polymérisation radicalaire des monomères de vinylimidazole de structure suivante : 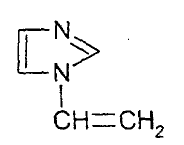
   Les copolymères peuvent être par exemple des copolymères de vinylimidazole comportant au moins 5% de motifs vinylimidazole avec des monomères choisis parmi les motifs : vinylpyrrolidinone, acide acrylique et acrylamide. La synthèse de tels composés est bien connue de l'homme du métier ; à ce sujet, on peut en particulier se reporter aux documents : J.Am.Chem.Soc., vol.85, 1962, p.951 ; Polymer Letters Ed., vol.11, 1973, p.465-469 ; Macromolecules, vol.6(2), 1973, p.163-168 ; Ann.N.Y.Acad.Sci., vol.155, 1969, p.431; FR-A-1,477,147 ; JP-69 07395 ; J.Macromol.Scien.Chem., vol.A21(2), 1984, p.253.
(C) le terme polyvinylpyridine comprend les homopolymères et les copolymères de vinyl pyridine obtenus par polymérisation radicalaire des monomères de vinyl pyridine (substitués en position 2 ou 4 du noyau pyridine) de structure suivante : 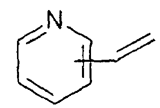
   Les copolymères peuvent être par exemple des copolymères de vinylpyridine comportant au moins 5% de motifs vinylpyridine avec des monomères choisis parmi les motifs : vinylpyrrolidinone, acide acrylique et acrylamide.
(D) Les produits d'addition de monomères 1-vinylimidazole répondant à la formule (I): 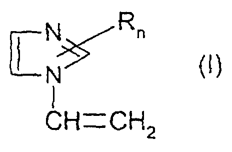 dans laquelle les radicaux R, identiques ou différents, représentent H, ou un radical alkyle en C₁-C₆, saturé ou insaturé, linéaire ou cyclique,
   n est un entier allant de 1 à 3
   avec les polyalkylènepolyamines et leurs dérivés (A)(i) à (A)(ix).
   Parmi les dérivés de formule (I) utilisables on peut citer par exemple le 2-méthyl 1-vinyl imidazole, le 2-benzyl 1-vinyl imidazole.
   Ces produits sont connus de l'homme du métier : Leur préparation est décrite par exemple dans la demande de brevet WO 94/29422 dont le contenu est incorporé à la présente demande par référence.
(E) Les dérivés réticulés des polymères (A)(i) à (A)(ix), (B), (C) et (D). Parmi les réticulants utilisables, on peut citer les dérivés halo- hydrin-, glycidyl, aziridino-, isocyanate ; de tels réticulants ainsi que leurs méthodes d'utilisation sont bien connus de l'homme du métier. Parmi les plus connus on peut citer: l'épichlorhydrine, les α,ω-bis-(chlorhydrin) polyalkylène glycoléthers, les α,ω-dichloroalkane comme par exemple le 1,2-dichloroéthane, le 1,2-dichloropropane, le 1,3-dichloropropane, le 1,4-dichlorobutane, et le 1,6-dichlorohexane ; de tels réticulants et leur utilisation pour réticuler des dérivés de polyéthylène imine sont décrits dans WO 94/12560 dont le contenu est incorporé à la présente demande par référence.

De préférence, on choisit dans la mise en oeuvre de la présente invention des polymères polyaminés comprenant au moins 5% d'amines tertiaires, avantageusement au moins 10% de fonctions amines tertiaires, et encore plus préférentiellement au moins 20%.

Selon l'invention, le polymère polyaminé est choisi avantageusement parmi :
(A)
   (i) les polyéthylèneimines hyperbranchées,
   (ii) les dérivés alkylés de polyéthylèneimine;
   (iii) les produits d'addition d'acides alkylcarboxyliques sur la polyéthylèneimine ;
   (iv) les produits d'addition de cétones et d'aldéhydes sur la polyéthylèneimine;
   (v) les produits d'addition d'isocyanates et d'isothiocyanates sur la polyéthylèneimine ;
   (vi) les produits d'addition d'oxyde d'alkylène ou de polymères blocs polyoxyde d'alkylène sur la polyéthylèneimine;
   (vii) les dérivés quatemisés de la polyéthylèneimine ;
   (viii) les produits d'addition d'une silicone sur les polyéthylèneimine ;
   (ix) les copolymères d'acide dicarboxylique et de polyéthylèneimine ;
(B) les polyvinylimidazoles

Encore plus préférentiellement, le polymère polyaminé est choisi parmi :
(A) (i) les polyéthylèneimines hyperbranchées. De préférence, on choisit des polyéthylèneimines comprenant au moins 5% d'amines tertiaires, avantageusement au moins 10% de fonctions amines tertiaires, et encore plus préférentiellement au moins 20%.

L'homme du métier saura, par de simples essais, adapter la proportion relative de rétinoïde et de polymère polyaminé pour obtenir l'effet recherché. En effet, les proportions optimales des différents constituants peuvent varier, par exemple en fonction du poids moléculaire du polymère, du taux d'amines et/ou du taux d'amines tertiaires dans ce polymère.

Outre le rétinoïde et le polymère polyaminé, l'association selon l'invention comprend avantageusement au moins un filtre solaire. L'adjonction d'un filtre solaire permet de renforcer la stabilité de l'association du rétinoïde avec le polymère polyaminé, en limitant l'action néfaste des UV sur le rétinoïde. Un tel constituant peut être choisi parmi les familles connues de filtres solaires actifs dans l'UVA et/ou l'UVB, hydrophiles ou lipophiles. On peut citer par exemple : les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre, les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β -diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665, les filtres hydrophiles contenant au moins un radical sulfonique -SO₃H, comme par exemple l'acide 2-phénylbenzimidazole 5-sulfonique ou l'acide benzène 1,4-di(3-méthylidène-10-camphosulfonique.

On peut également incorporer à l'association selon l'invention un autre composé connu de l'art antérieur comme stabilisateur du rétinol. Parmi ces composés on peut citer : les agents chélatants, et les polysaccharides, les huiles ayant un indice d'iode supérieur ou égal à 70, les polyéthylène glycol et/ou les polypropylène glycols, les hydroxycarboxylates, les acides aminés et leurs sels, les anti-oxydants tels que le butyl hydroxytoluène, le butyl hydroxyanisole, les α, β, γ, δ-tocophérols, la nordihydrogaiaretine, le gallate de propyle, les esters d'acides gras et de vitamine C, l'acide ascorbique, les sels d'acide ascorbique, l'acide iso-ascorbique, les sels d'acide iso-ascorbique, l'acide sorbique, les sels d'acide sorbique, le butanediol, les esters d'acide gras et de pentaérythritol, les esters gras de triméthylolpropane, les argiles minérales hydrophiles. De tels composés sont connus comme des stabilisants des rétinoïdes, on peut à ce sujet se reporter au document : EP-A-608433.

Dans le cas particulier du traitement de l'acné, on peut également incorporer dans l'association selon l'invention, de façon avantageuse, au moins un agent antiacnéïque spécifique, antiséborrhéique et/ou antibactérien, et en particulier, la piroctone olamine, commercialisée sous la dénomination Octopirox par la société HOECHST.

Dans les compositions selon l'invention, le rétinoïde est de préférence introduit en une quantité allant de 0,0001 à 10% en poids par rapport au poids total de la composition, avantageusement de 0,001 à 3%, encore plus préférentiellement de 0,01 à 1%.

Dans les compositions selon l'invention, le polymère polyaminé est de préférence introduit en une quantité allant de 0,001 à 20% en poids par rapport au poids total de la composition, avantageusement de 0,1 à 10%.

Les compositions de l'invention peuvent comprendre en outre des adjuvants cosmétiques ou dermatologiques choisis parmi les corps gras, les solvants organiques, les émulsionnants, les épaississants non ioniques, les adoucissants, les antioxydants, les opacifiants, les stabilisants, les silicones, les agents anti-mousse, les agents hydratants, les vitamines, les parfums, les conservateurs, les tensioactifs, de préférence non ioniques, les charges, les séquestrants, les polymères autres que ceux décrits ci-dessus, les propulseurs, les agents alcalinisants ou acidifiants, les colorants, ou tout autre ingrédient habituellement utilisé en cosmétique.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en C₁₂-C₁₅ (Finsolv TN de FINETEX), l'alcool myristique polyoxypropyléné à 3 moles d'oxyde de propylène (WITCONOL APM de WITCO), les triglycérides d'acides gras en C₆-C₁₈ tels que les triglycérides d'acide caprylique/caprique.

On peut citer parmi les huiles utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide des huiles d'hydrocarbures telles que des huiles de paraffine, le squalane, la vaseline; des esters tels que le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées; des huiles perfluorées et/ou organofluorées ;des acides gras supérieurs tels que l'acide myristique, l'acide caprique, l'acide caprylique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique.

Les cires peuvent être choisies parmi les cires animales, végétales, minérales ou synthétiques. Parmi les cires animales, on peut notamment citer les cires d'abeilles, la cire de baleine. Parmi les cires végétales, on peut citer, entre autres, les cires de Carnauba, de Candellila, d'Ouricoury, les cires de fibres de liège, les cires de canne à sucre et les cires du Japon. Parmi les cires minérales, on peut citer, en particulier, les cires de paraffine, la lanoline, les cires microcristallines, les cires de lignite et les ozokérites. Parmi les cires synthétiques, on peut citer, notamment, les cires de polyéthylène et les cires obtenues par synthèse de Fisher et Tropsch. Toutes ces cires sont bien connues de l'Homme de l'Art.

Parmi les solvants organiques, on peut citer les alcools et polyols inférieurs tels que l'éthanol, l'isopropanol, le propylèneglycol, la glycérine et le sorbitol.

Les épaississants, de préférence non ioniques, peuvent être choisis parmi les gommes de guar et celluloses modifiées ou non telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Les compositions selon l'invention peuvent se présenter sous la forme d'une lotion, d'un gel, d'une émulsion eau-dans-huile, d'une émulsion huile-dans-eau, d'une émulsion triple. Elles peuvent également se trouver sous une forme vectorisée, comme par exemple sous forme de nanocapsules, de liposomes, de nanoémulsions, d'oléosomes.

Ces compositions sont destinées à une application sur la peau du corps, en particulier la peau du visage et des mains, sur les muqueuses et les semi-muqueuses, ou encore sur les cheveux.

De telles compositions peuvent être sous la forme de produits de soin ou de maquillage. En particulier elles peuvent se présenter sous la forme d'une crème de soin, d'un lait, d'un tonique, d'un produit nettoyant et/ou démaquillant, d'un masque, d'un produit de gommage, d'un exfoliant, d'un produit de protection solaire, d'un fond de teint, d'une crème teintée, d'un rouge-à-lèvres.

D'autres caractéristiques et avantages de la composition de l'invention ressortiront mieux des exemples et contre-exemples qui suivent et qui sont donnés à titre illustratif et non limitatif. Dans ces exemples et contre-exemples, les pourcentages sont donnés en poids de constituant par rapport au volume de solvant (W/V), sauf indication contraire.

### EXEMPLES :

Dans les essais décrits ci-dessous, nous avons utilisé la polyéthylèneimine (PEI) de poids moléculaire 700 commercialisée par la société Aldrich.

### Test de stabilité :

La stabilité d'un rétinoïde est définie conformément à la présente invention par le pourcentage du rétinoïde qui est resté sous sa forme d'origine après que la composition le comprenant ait été stockée pendant un temps donné et à une température donnée.

Une composition destinée à un usage commercial devrait avoir une stabilité d'au moins 80 % de la forme active du rétinoïde qu'elle contient après 20 jours de stockage à 50° C.

### ESSAIS :

### Test de stabilité du rétinol all trans à 0.3% dans l'éthanol en conservation à 50°C

Les conditions de l'étude sont les suivantes :
- PEI à 0.25, 0.5, et 1% (en poids/volume) dans l'éthanol
- Rétinol all trans commercialisé par la société Fluka à 0.3% (en poids/volume) dans l'éthanol
- Milieu aérobie avec 55% de volume d'air, 45% volume d'éthanol
- Conservation à 50°C à l'étuve

La dégradation du rétinol dans ces conditions atteint près de 99% en 20 jours de conservation à 50°C. Les conditions de conservation auxquelles nous avons soumis les compositions sont particulièrement sévères.

On teste dans ces conditions les associations suivantes :

| **Essai 1 : Mesure de l'effet dose de la PEI** | | | | |
|---|---|---|---|---|
| Constituants | Formule A1 | Formule B1 | Formule C1 | Formule D1 |
| Rétinol all trans fluka (W/V) | 0.3% | 0.3% | 0.3% | 0.3% |
| PEI-700 aldrich (W/V) | - | 0.25% | 0.5% | 1% |
| Ethanol Absolu en ml | 100 | 100 | 100 | 100 |

On suit par HPLC (sur colonne Sphérisorb ODS1 C18, par élution avec un mélange H₂O/CH₃CO₂H/CH₃CO₂NH₄/Acétonitrile: 15/1/0,4/83,6) le taux de rétinol résiduel après un mois de conservation à 50°C en milieu aérobie (55% de volume d'air).

On obtient les résultats suivants :

| Formule | Taux de rétinol résiduel |
|---|---|
| Formule A1 | 0% |
| Formule B1 | 88% |
| Formule C1 | 90% |
| Formule D1 | 91% |

| **Essai 2 : Comparaison PEI, butyl hydroxytoluène (BHT), et tocophérols** | | | | |
|---|---|---|---|---|
| constituants | Formule A2 | Formule B2 | Formule C2 | Formule D2 |
| Rétinol all trans fluka (W/V) | 0.3% | 0.3% | 0.3% | 0.3% |
| PEI-700 aldrich (W/V) | - | 0.25% | - | - |
| BHT(W/V) | - | - | 0.25% | - |
| α-tocophérol (W/V) | - | - | - | 0.25% |
| Ethanol Absolu en ml | 100 | 100 | 100 | 100 |

On suit par HPLC (mêmes conditions qu'à l'essai 1) le taux de rétinol résiduel après deux mois de conservation à 50°C en milieu aérobie (55% de volume d'air).

On obtient les résultats suivants :

| Formule | Taux de rétinol résiduel |
|---|---|
| Formule A2 | 0% |
| Formule B2 | 83% |
| Formule C2 | 17% |
| Formule D2 | 27% |

| **Essai 3 : Influence de la neutralisation de la PEI par l'acide acétique** | | | |
|---|---|---|---|
| Constituants | Formule A3 | Formule B3 | Formule C3 |
| Rétinol all trans fluka (W/V) | 0.3% | 0.3% | 0.3% |
| PEI-700 aldrich (W/V) | - | 1% | 1% |
| Acide acétique (W/V) | - | - | 1% |
| Ethanol Absolu en ml | 100 | 100 | 100 |

On suit par HPLC (mêmes conditions qu'à l'essai 1) le taux de rétinol résiduel après un mois de conservation à 50°C en milieu aérobie (55% de volume d'air).

On obtient les résultats suivants :

| Formule | Taux de rétinol résiduel |
|---|---|
| Formule A3 | 0% |
| Formule B3 | 91% |
| Formule C3 | 89% |

On constate par cet essais que la polyéthylèneimine conserve son activité de stabilisateur de rétinoïde quel que soit le pH du milieu.

| **Essai 4 : Comparaison PEI et vitamine C :** | | |
|---|---|---|
| Constituants | Formule A4 | Formule B4 |
| Rétinol all trans fluka (W/V) | 0.26% | 0.26% |
| PEI-700 aldrich (W/V) | 0,1% | - |
| Acide ascorbique (W/V) | - | 0,25% |
| Ethanol Absolu en ml | 100 | 100 |

A partir d'une solution alcoolique de rétinol à 0,26%, on suit par HPLC le taux de rétinol résiduel après plusieurs jours de stockage des formules à 50°C en milieu aérobie (55% de volume d'air).

On obtient les résultats suivants après 7 jours de stockage :

| Formule | Taux de rétinol résiduel |
|---|---|
| Formule A4 | 0,26% |
| Formule B4 | 0% |

| **Essai 5 : Comparaison PEI et EDTA :** | | |
|---|---|---|
| Constituants | Formule A5 | Formule B5 |
| Rétinol all trans fluka (W/V) | 0.26% | 0.26% |
| PEI-700 aldrich (W/V) | 0,1% | - |
| EDTA (W/V) | - | 0,1% |
| Ethanol Absolu en ml | 100 | 100 |

A partir d'une solution alcoolique de rétinol à 0,26%, on suit par HPLC le taux de rétinol résiduel après plusieurs jours de stockage des formules à 50°C en milieu aérobie (55% de volume d'air). On obtient les résultats suivants après 1, 2 et 5 jours de stockage :

| | 1 jour | 2 jours | 5 jours |
|---|---|---|---|
| Formule A5 | 0,23 % | 0,23 % | 0,22 % |
| Formule B5 | 0,08% | 0,025% | 0% |

| **Essai 6 : Comparaison PEI, spermine et spermidine :** | | | |
|---|---|---|---|
| Constituants | Formule A6 | Formule B6 | Formule C6 |
| Rétinol all trans fluka (W/V) | 0,3% | 0,3% | 0,3% |
| PEI-700 aldrich (W/V) (*) | 0,25% | - | - |
| spermine (*) | - | 0,25% | - |
| spermidine (*) | - | - | 0,25% |
| Ethanol Absolu en ml | 100 | 100 | 100 |

| | | | |
|---|---|---|---|
| (*) composé neutralisé à 75% par de l'acide acétique | | | |

A partir d'une solution alcoolique de rétinol à 0,3%, on mesure par HPLC le taux de rétinol résiduel après plusieurs jours de stockage des formules à 50°C en milieu aérobie (55% de volume d'air). Cette mesure est convertie en taux de dégradation du rétinol :
% dégradation = ([rétinol (T₀)]-[rétinol (Tₓ)])/[rétinol (T₀)].
[rétinol (T₀)] désigne la concentration initiale en rétinol dans la solution
[rétinol (Tₓ)] désigne la concentration en rétinol dans la solution au temps Tₓ

On obtient les résultats suivants après 4, 14 et 21 jours de stockage :

| | 4 jours | 14 jours | 21 jours |
|---|---|---|---|
| Formule A6 | 1 % | 4 % | 7 % |
| Formule B6 | 12 % | 32 % | 41 % |
| Formule C6 | 16% | 40 % | 50 % |

L'ensemble des ces essais montre la supériorité de la polyéthylèneimine dans la stabilisation du rétinol, par rapport aux produits connus jusqu'alors comme agents anti-oxydants ou comme stabilisateurs de rétinoïdes.

## Revendications

1. Composition cosmétique et/ou dermatologique adaptée à une application sur la peau du corps ou les cheveux, comprenant un support physiologiquement acceptable et au moins un rétinoïde choisi parmi la vitamine A et les précurseurs bio-convertibles de la vitamine A, **caractérisée par le fait qu'**elle comprend au moins un polymère polyaminé choisi parmi :
(A) une polyalkylène polyamine ou un de ses dérivés choisi parmi :
(i) les polyalkylène polyamines ;
(ii) les dérivés alkylés de polyalkylène polyamines ;
(iii) les produits d'addition d'acides alkylcarboxyliques sur les polyalkylènepolyamines ;
(iv) les produits d'addition de cétones et d'aldéhydes sur les polyalkylènepolyamines ;
(v) les produits d'addition d'isocyanates et d'isothiocyanates sur les polyalkylènepolyamines ;
(vi) les produits d'addition d'oxyde d'alkylène ou de polymères blocs polyoxyde d'alkylène sur les polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;
(B) les polyorinylimidazoles ;
(C) les polyvinylpyridines ;
(D) les produits d'addition de monomères 1-vinylimidazole de formule (I): 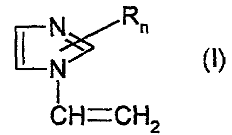 dans laquelle les radicaux R, identiques ou différents, représentent H, ou un radical alkyle en C₁-C₆, saturé ou insaturé, linéaire ou cyclique
n est un entier allant de 1 à 3,
avec les polyalkylènepolyamines (A)(i) à (A)(ix) ;
(E) les dérivés réticulés des polymères (A)(i) à (A)(ix), (B) (C) et (D).

2. Composition selon la revendication 1, **caractérisée par le fait que** le rétinoïde est choisi parmi le rétinol de type all-trans et le rétinol de type 13-cis.

3. Composition selon la revendication 1, **caractérisée par le fait que** le rétinoïde est choisi parmi les esters de rétinol.

4. Composition selon la revendication 3, **caractérisée par le fait que** le rétinoïde est choisi parmi les esters de rétinol C₁-C₆.

5. Composition selon l'une quelconque des revendications précédentes, **caractérisée par le fait que** le polymère polyaminé est choisi parmi ceux comprenant au moins 5% de fonctions amines tertiaires, avantageusement au moins 10% de fonctions amines tertiaires, et encore plus préférentiellement au moins 20%.

6. Composition selon l'une quelconque des revendications précédentes, **caractérisée par le fait que** le polymère polyamine est choisi parmi :
(A)
(i) les polyéthylèneimines hyperbranchées,
(ii) les dérivés quatemisés de polyéthylèneimine ;
(iii) les produits d'addition d'acides alkylcarboxyliques sur la polyéthylèneimine ;
(iv) les produits d'addition de cétones et d'aldéhydes sur la polyéthylèneimine ;
(v) les produits d'addition d'isocyanates et de thioisocyanates sur la polyéthylèneimine ;
(vi) les produits d'addition d'oxyde d'alkylène ou de polymères blocs polyoxyde d'alkylène sur la polyéthylèneimine ;
(vii) les dérivés quatemisés de polyéthylèneimine ;
(viii) les produits d'addition d'une silicone sur la polyéthylèneimine ;
(ix) un copolymère d'acide dicarboxylique et de polyéthylèneimine ;
(B) les polyvinylimidazoles.

7. Composition selon l'une quelconque des revendications précédentes, **caractérisée par le fait que** le polymère polyaminé est choisi parmi les polyéthylèneimines hyperbranchées.

8. Composition selon l'une quelconque des revendications précédentes, **caractérisée par le fait qu'**elle comprend en outre au moins un filtre solaire.

9. Composition selon l'une quelconque des revendications 1 à 8, **caractérisée par le fait que** le rétinoïde est présent en une quantité allant de 0,0001 à 10% en poids par rapport au poids total de la composition, avantageusement de 0,001 à 3%, encore plus préférentiellement de 0,01 à 1%.

10. Composition selon l'une quelconque des revendications 1 à 9, **caractérisée par le fait que** le polymère polyaminé est présent en une quantité allant de 0,001 à 20% en poids par rapport au poids total de la composition, avantageusement de 0,1 à 10%.

11. Composition selon l'une quelconque des revendications 1 à 10, **caractérisée par le fait qu'**elle comprend en outre au moins un composé choisi parmi : les agents chélatant, les polysaccharides, les huiles ayant un indice d'iode supérieur ou égal à 70, les polyéthylène glycol et/ou les polypropylène glycols, les hydroxycarboxylates, les acides aminés et leurs sels, les anti-oxydants tels que le butyl hydroxytoluène, le butyl hydroxyanisole, les α, β, γ, δ-tocophérols, la nordihydrogaiaretine, le gallate de propyle, les esters d'acides gras et de vitamine C, l'acide ascorbique, les sels d'acide ascorbique, l'acide iso-ascorbique, les sels d'acide iso-ascorbique, l'acide sorbique, les sels d'acide sorbique, le butanediol, les esters d'acide gras et de pentaérythritol, les esters gras de triméthylolpropane, les argiles minérales hydrophiles.

12. Composition selon l'une quelconque des revendications 1 à 11, **caractérisée par le fait qu'**elle comprend en outre au moins un agent antiacnéïque spécifique, antiséborrhéique et/ou antibactérien.

13. Composition selon l'une quelconque des revendications 1 à 12, **caractérisée par le fait qu'**elle est sous la forme d'une lotion, d'un gel, d'une émulsion eau-dans-huile, d'une émulsion huile-dans-eau, d'une émulsion triple, de nanocapsules, de liposomes, de nanoémulsions, d'oléosomes.

14. Composition selon l'une quelconque des revendications 1 à 13, **caractérisée par le fait qu'**elle est sous la forme d'une crème de soin, d'un lait, d'un tonique, d'un produit nettoyant et/ou démaquillant, d'un masque, d'un produit de gommage, d'un exfoliant, d'un produit de protection solaire, d'un fond de teint, d'une crème teintée, d'un rouge-à-lèvres.

15. Utilisation d'une composition selon l'une quelconque des revendications 1 à 14 pour la fabrication d'une préparation destinée au traitement dermatologique de lutte contre, et/ou de prévention de, l'irritation, l'inflammation, l'immunosuppression et/ou l'acné.

16. Procédé de traitement cosmétique consistant à lutter contre les signes du viellissement, plus particulièrement les signes du vieillissement induits par la photoperoxydation, en particulier la photoperoxydation du squalène et/ou du collagène par application topique sur la peau et/ou le cuir chevelu et/ou les cheveux d'une composition selon l'une quelconque des revendications 1 à 14, les méthodes de traitement thérapeutique du corps humain étant exclues.

17. Utilisation d'une composition cosmétique selon l'une quelconque des revendications 1 à 14 pour lutter contre et/ou prévenir les signes du vieillissement cutané ou des cheveux, les méthodes de traitement thérapeutique du corps humain étant exclues.

18. Utilisation selon la revendication 17 pour lutter contre et/ou prévenir les signes du vieillissement photoinduits de la peau et/ou des cheveux.

## Patentansprüche

1. Kosmetische und/oder dermatologische Zusammensetzung, die auf die Haut oder die Haare aufgetragen werden kann und ein physiologisch akzeptables Medium und mindestens ein Retinoid enthält, das unter Vitamin A und den biologisch umwandelbaren Precursoren von Vitamin A ausgewählt ist, **dadurch gekennzeichnet, daß** sie mindestes ein Polyaminpolymer enthält, das ausgewählt ist unter:
(A) den Polyalkylenpolyaminen oder Polyalkylenpolyaminderivaten, die ausgewählt sind unter:
(i) den Polyalkylenpolyaminen;
(ii) den Alkylderivaten von Polyalkylenpolyaminen;
(iii) den Additionsprodukten von Alkylcarbonsäuren und Polyalkylenpolyaminen;
(iv) den Additionsprodukten von Ketonen und Aldehyden mit Polyalkylenpolyaminen;
(v) den Additionsprodukten von Isocyanaten und Isothiocyanaten und Polyalkylenpolyaminen;
(vi) den Additionsprodukten von Alkylenoxid oder Polyalkylenoxid-Blockpolymeren mit Polyalkylenpolyaminen;
(vii) den quaternisierten Derivaten von Polyalkylenpolyaminen;
(viii) den Additionsprodukten von Siliconen und Polyalkylenpolyaminen;
(ix) den Copolymeren von Dicarbonsäuren und Polyalkylenpolyaminen;
(B) den Polyvinylimidazolen;
(C) den Polyvinylpyridinen;
(D) den Additionsprodukten von 1-Vinylimidazolmonomeren der Formel (1): 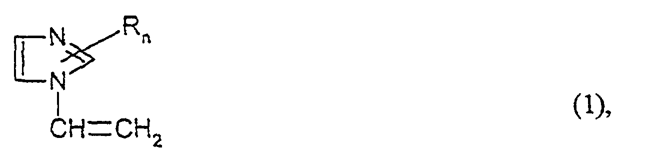 worin die Gruppen R, die identisch oder voneinander verschieden sind, H oder eine geradkettige oder cyclische, gesättigte oder ungesättigte C₁₋₆-Alkylgruppe und n eine ganze Zahl von 1 bis 3 bedeuten, und den Polyalkylenpolyaminen (A)(i) bis (A)(ix); und
(E) den vernetzten Derivaten von Polymeren (A)(i) bis (A)(ix), (B), (C) und (D).

2. Zusammensetzung nach Anspruch 1, **dadurch gekennzeichnet, daß** das Retinoid unter Retinol vom Typ all-trans oder Retinol vom Typ 13-cis ausgewählt ist.

3. Zusammensetzung nach Anspruch 1, **dadurch gekennzeichnet, daß** das Retinoid unter den Retinolestern ausgewählt ist.

4. Zusammensetzung nach Anspruch 3, **dadurch gekennzeichnet, daß** das Retinoid unter den C₁₋₆-Retinolestern ausgewählt ist.

5. Zusammensetzung nach einem der vorhergehenden Ansprüche, **dadurch gekennzeichnet, daß** das Polyaminpolymer unter den Polymeren ausgewählt ist, die mindestens 5 % tertiäre Aminogruppen, vorteilhaft mindestens 10 % tertiäre Aminogruppen und noch bevorzugter mindestens 20 % tertiäre Aminogruppen enthalten.

6. Zusammensetzung nach einem der vorhergehenden Ansprüche, **dadurch gekennzeichnet, daß** das Polyaminpolymer ausgewählt ist unter:
(A)
(i) den hochverzweigten Polyethyleniminen;
(ii) den quaternisierten Polyethyleniminderivaten;
(iii) den Additionsprodukten von Alkylcarbonsäuren und Polyethylenimin;
(iv) den Additionsprodukten von Ketonen und Aldehyden und Polyethylenimin;
(v) den Additionsprodukten von Isocyanaten und Isothiocyanaten und Polyethylenimin;
(vi) den Additionsprodukten von Alkylenoxid oder Blockpolymeren von Polyalkylenoxid und Polyethylenimin;
(vii) den quaternisierten Derivaten von Polyethylenimin;
(viii) den Additionsprodukten von Siliconen und Polyethylenimin;
(ix) den Copolymeren von Dicarbonsäuren und Polyethylenimin;
(B) den Polyvinylimidazolen.

7. Zusammensetzung nach einem der vorhergehenden Ansprüche, **dadurch gekennzeichnet, daß** das Polyaminpolymer unter den hochverzweigten Polyethyleniminen ausgewählt ist.

8. Zusammensetzung nach einem der vorhergehenden Ansprüche, **dadurch gekennzeichnet, daß** sie ferner mindestens ein Sonnenschutzfilter enthält.

9. Zusammensetzung nach einem der Ansprüche 1 bis 8, **dadurch gekennzeichnet, daß** das Retinoid in Mengenanteilen im Bereich von 0,0001 bis 10 Gew.-%, vorteilhaft 0,001 bis 3 Gew.-% und noch bevorzugter 0,01 bis 1 Gew.-%, bezogen auf das Gesamtgewicht der Zusammensetzung, vorliegt.

10. Zusammensetzung nach einem der Ansprüche 1 bis 9, **dadurch gekennzeichnet, daß** das Polyaminpolymer in Mengenanteilen im Bereich von 0,001 bis 20 Gew.-% und vorteilhaft 0,1 bis 10 Gew.-%, bezogen auf das Gesamtgewicht der Zusammensetzung, vorliegt.

11. Zusammensetzung nach einem der Ansprüche 1 bis 10, **dadurch gekennzeichnet, daß** sie ferner mindestens eine Verbindung enthält, die ausgewählt ist unter: Chelatbildnern, Polysacchariden, Ölen, die einen Iodindex von mindestens 70 aufweisen, Polyethylenglykolen und/oder Polypropylenglykolen, Hydroxycarboxylaten, Aminosäuren und ihren Salzen, Antioxidantien, wie Butylhydroxytoluol, Butylhydroxyanisol, α-, β-, γ- und δ-Tocopherol, Nordihydrogaiaretin, Propylgallat, Estern von Fettsäuren und Vitamin C, Ascorbinsäure, Salzen von Ascorbinsäure, Isoascorbinsäure, Salzen von Isoascorbinsäure, Sorbinsäure, Salzen von Sorbinsäure, Butandiol, Estern von Fettsäuren und Pentaerythrit, Fettsäurestern von Trimethylolpropan und hydrophilen anorganischen Tonen.

12. Zusammensetzung nach einem der Ansprüche 1 bis 11, **dadurch gekennzeichnet, daß** sie ferner mindestens ein spezielles Mittel gegen Akne, gegen Seborrhoe und/oder gegen Bakterien enthält.

13. Zusammensetzung nach einem der Ansprüche 1 bis 12, **dadurch gekennzeichnet, daß** sie als Lotion, Gel, Wasser-in-Öl-Emulsion, Öl-in-Wasser-Emulsion, dreifache Emulsion, Nanokapseln, Liposomen, Nanoemulsionen oder Oleosomen vorliegt.

14. Zusammensetzung nach einem der Ansprüche 1 bis 13, **dadurch gekennzeichnet, daß** sie als Creme zur Pflege, Milch, Tonikum, Produkt zum Reinigen und/oder Abschminken, Maske, abradierendes Produkt, Exfoliationsmittel, Produkt zum Sonnenschutz, Make-up, getönte Creme oder Lippenstift vorliegt.

15. Verwendung einer Zusammensetzung nach einem der Ansprüche 1 bis 14 zur Herstellung eines Präparats, das zur dermatologischen Bekämpfung und/oder Vorbeugung von Reizungen, Entzündungen, Immunosuppression und/oder Akne vorgesehen ist.

16. Verfahren zur kosmetischen Behandlung, das darin besteht, die Anzeichen der Alterung, insbesondere die durch Photoperoxidation und ganz besonders die durch Photoperoxidation von Squalen und/oder Kollagen hervorgerufenen Anzeichen der Alterung zu bekämpfen, indem auf die Haut und/oder die Kopfhaut und/oder die Haare topisch eine Zusammensetzung nach einem der Ansprüche 1 bis 14 aufgebracht wird, wobei Verfahren zur therapeutischen Behandlung des menschlichen Körpers ausgenommen sind.

17. Verwendung einer kosmetischen Zusammensetzung nach einem der vorhergehenden Ansprüche 1 bis 14, um die Anzeichen der Alterung der Haut oder der Haare zu bekämpfen und/oder ihnen vorzubeugen, wobei Verfahren zur therapeutischen Behandlung des menschlichen Körpers ausgenommen sind.

18. Verwendung nach Anspruch 17, um die durch Licht hervorgerufenen Anzeichen der Alterung der Haut und/oder der Haare zu bekämpfen und/oder ihnen vorzubeugen.

## Claims

1. Cosmetic and/or dermatological composition suitable for application to the skin of the body or the hair, comprising a physiologically acceptable carrier and at least one retinoid chosen from vitamin A and the bioconvertible precursors of vitamin A, **characterized**
**in that** it comprises at least one polyamine polymer chosen from:
(A) a polyalkylenepolyamine or one of its derivatives chosen from:
(i) polyalkylenepolyamines;
(ii) alkylated derivatives of polyalkylenepolyamines;
(iii) products of addition of alkylcarboxylic acids to the polyalkylenepolyamines;
(iv) products of addition of ketones and of aldehydes to the polyalkylenepolyamines;
(v) products of addition of isocyanates and of isothiocyanates to the polyalkylenepolyamines;
(vi) products of addition of alkylene oxide or of polyalkylene oxide block polymers to the polyalkylenepolyamines;
(vii) quaternized derivatives of polyalkylenepolyamines;
(viii) products of addition of a silicone to the polyalkylenepolyamines;
(ix) a copolymer of dicarboxylic acid and polyalkylenepolyamines ;
(B) polyvinylimidazoles;
(C) polyvinylpyridines;
(D) products of addition of 1-vinylimidazole monomers of formula (I) : 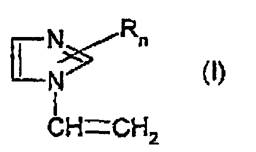 in which the R radicals, which are identical or different, represent H, or a linear or cyclic, saturated or unsaturated C₁-C₆ alkyl radical
n is an integer ranging from 1 to 3,
to the polyalkylenepolyamines (A) (i) to (A)(ix);
(E) cross-linked derivatives of the polymers (A)(i) to (A)(ix), (B) (C) and (D).

2. Composition according to Claim 1, **characterized in that** the retinoid is chosen from retinol of the alltrans type and retinol of the 13-cis type.

3. Composition according to Claim 1, **characterized**
**in that** the retinoid is chosen from retinol esters.

4. Composition according to Claim 3, **characterized in that** the retinoid is chosen from C₁-C₆ retinol esters.

5. Composition according to any one of the preceding claims, **characterized in that** the polyamine polymer is chosen from those comprising at least 5% of tertiary amine functional groups, advantageously at least 10% of tertiary amine functional groups, and still more preferably at least 20%.

6. Composition according to any one of the preceding claims, **characterized in that** the polyamine polymer is chosen from:
(A)
(i) hyperbranched polyethyleneimines,
(ii) quaternized derivatives of polyethyleneimine;
(iii) products of addition of alkylcarboxylic acids to polyethyleneimine;
(iv) products of addition of ketones and of aldehydes to polyethyleneimine;
(v) products of addition of isocyanates and of thioisocyanates to polyethyleneimine;
(vi) products of addition of alkylene oxide or of polyalkylene oxide block polymers to polyethyleneimine;
(vii) quaternized derivatives of polyethyleneimine;
(viii) products of addition of a silicone to polyethyleneimine;
(ix) a copolymer of dicarboxylic acid and of polyethyleneimine;
(B)polyvinylimidazoles.

7. Composition according to any one of the preceding claims, **characterized in that** the polyamine polymer is chosen from hyperbranched polyethyleneimines.

8. Composition according to any one of the preceding claims, **characterized in that** it comprises, in addition, at least one sunscreening agent.

9. Composition according to any one of Claims 1 to 8, **characterized in that** the retinoid is present in a quantity ranging from 0.0001 to 10% by weight relative to the total weight of the composition, advantageously from 0.001 to 3%, still more preferably from 0.01 to 1%.

10. Composition according to any one of Claims 1 to 9, **characterized in that** the polyamine polymer is present in a quantity ranging from 0.001 to 20% by weight relative to the total weight of the composition, advantageously from 0.1 to 10%.

11. Composition according to any one of Claims 1 to 10, **characterized in that** it comprises, in addition, at least one compound chosen from: chelating agents, polysaccharides, oils having an iodine number greater than or equal to 70, polyethylene glycol and/or polypropylene glycols, hydroxycarboxylates, amino acids and their salts, antioxidants such as butylated hydroxytoluene, butylated hydroxyanisole, α-, β-, γ-and δ-tocopherols, nordihydrogaiaretin, propyl gallate, esters of fatty acids and of vitamin C, ascorbic acid, ascorbic acid salts, isoascorbic acid, isoascorbic acid salts, sorbic acid, sorbic acid salts, butanediol, fatty acid and pentaerythritol esters, fatty esters of trimethylolpropane, and hydrophilic mineral clays.

12. Composition according to any one of Claims 1 to 11, **characterized in that** it comprises, in addition, at least one specific anti-acne, antiseborrhoeic and/or antibacterial agent.

13. Composition according to any one of Claims 1 to 12, **characterized in that** it is in the form of a lotion, a gel, a water-in-oil emulsion, an oil-in-water emulsion, a triple emulsion, nanocapsules, liposomes, nanoemulsions or oleosomes.

14. Composition according to any one of Claims 1 to 13, **characterized in that** it is in the form of a care cream, a milk, a tonic, a cleansing and/or make-up removing product, a pack, a peeling product, an exfoliant, a sun protection product, a foundation, a tinted cream or a lipstick.

15. Use of a composition according to any one of Claims 1 to 14 for the manufacture of a preparation intended for dermatological treatment for combating and/or for preventing irritation, inflammation, immunosuppression and/or acne.

16. Process of cosmetic treatment consisting in combating the signs of ageing, more particularly the signs of ageing which are induced by photoperoxidation, in particular photoperoxidation of squalene and/or of collagen by topical application to the skin and/or the scalp and/or the hair of a composition according to any one of Claims 1 to 14, methods of therapeutic treatment of the human body being excluded.

17. Use of a cosmetic composition according to any one of Claims 1 to 14 for combating and/or preventing the signs of ageing of the skin or of the hair, methods of therapeutic treatment of the human body being excluded.

18. Use according to Claim 17, for combating and/or preventing the signs of photoinduced ageing of the skin and/or of the hair.
